# EUROPÄISCHE PATENTSCHRIFT

(11) **EP 3 117 448 B1**
(45) Veröffentlichungstag und Bekanntmachung des Hinweises auf die Patenterteilung: **02.12.2020**
(21) Anmeldenummer: 15715142.4
(22) Anmeldetag: 10.03.2015
(51) Int. Cl.: H01J 37/22, H01J 37/244

(54) **VORRICHTUNG FÜR DIE KORRELATIVE RASTER-TRANSMISSIONSELEKTRONENMIKROSKOPIE (STEM) UND LICHTMIKROSKOPIE**
DEVICE FOR CORRELATIVE SCANNING TRANSMISSION ELECTRON MICROSCOPY (STEM) AND LIGHT MICROSCOPY
DISPOSITIF DESTINÉ À LA MICROSCOPIE CORRÉLATIVE AU MOYEN D'UN MICROSCOPE ÉLECTRONIQUE À BALAYAGE EN TRANSMISSION (MEBT) ET D'UN MICROSCOPE OPTIQUE

(30) Priorität: 12.03.2014 DE 102014103360
(43) Veröffentlichungstag der Anmeldung: 18.01.2017
(73) Patentinhaber: Leibniz-Institut für Neue Materialien gemeinnützige GmbH, 66123 Saarbrücken (DE)
(72) Erfinder: DE JONGE, Niels, 66386 St. Ingbert (DE)
(74) Vertreter: Patentanwälte Gierlich & Pischitzis Partnerschaft mbB
(86) Internationale Anmeldenummer: PCT/DE2015/100097
(87) Internationale Veröffentlichungsnummer: WO 2015/135534

(56) Entgegenhaltungen:
- EP-A1- 2 924 706
- EP-A1- 2 975 631
- WO-A1-2010/120238
- US-A- 4 990 776
- US-A- 5 811 804
- US-A1- 2012 120 226

## Beschreibung

Die Erfindung betrifft eine Vorrichtung für die korrelative Raster-Transmissionselektronenmikroskopie (STEM) und Lichtmikroskopie.

Das Beobachten von Vorgängen, Zusammensetzungen und Strukturen biologischer Proben, die eine flüssige Phase enthalten, ist im Nanometerbereich schwierig durchzuführen. Derartige Proben können beispielsweise Proteinkomplexe in eukaryotischen Zellen, Lipidvesikeln in Lösung oder in Zellen oder Strukturen zellulären Materials sein. Die Lichtmikroskopie ermöglicht im Standardbereich Auflösungen von etwa 200 nm und von 20 bis 30 nm mittels spezieller Techniken. Die Röntgenmikroskopie ist insofern problematisch, als geeignete Röntgenquellen einen großen apparativen Aufwand erfordern. Rastersondenverfahren sind nur für Oberflächenstudien geeignet. Die Elektronenmikroskopie wird traditionell für Untersuchungen im Nanobereich verwendet, erfordert jedoch ein Vakuum für die Elektronenoptik, womit Untersuchungen von Proben in Flüssigkeit ausscheiden. In den letzten Jahren wurden daher Techniken entwickelt, um Elektronenmikroskopie in Flüssigkeiten durchzuführen. In der Regel wird die Flüssigkeit in einer Kammer mit einem dünnen und elektronendurchlässigen Fenster eingeschlossen. Von besonderem Vorteil ist die Verwendung von Raster-Transmissionselektronenmikroskopie (STEM; scanning transmission electron microscopy), um Auflösungen im Nanometerbereich von Nanopartikeln hoher Atomzahlen, beispielsweise Goldnanopartikeln, in dicken Schichten von Flüssigkeiten mit niedrigen Atomzahlen, beispielsweise Wasser, zu erreichen. Nanopartikel werden oft als spezifische Proteinlabels verwendet, um Zellprozesse zu untersuchen.

Obwohl in den meisten Fällen eine hohe Auflösung erreicht werden kann, ist der Probenraum auf einige Mikrometer beschränkt, wenn eine Auflösung von einigen Nanometern gewünscht wird.

Ein anderer Ansatz ist die Verwendung von atmosphärischer Rasterelektronenmikroskopie (ESEM; environmental scanning electron microscopy). Proben in einer Flüssigkeit, beispielsweise Wasser, werden auf einige Grad Celsius heruntergekühlt und dann in Wasserdampf bei niedrigem Druck aufbewahrt. Der Druck kann so eingestellt werden, daß ein Gleichgewicht zwischen der Flüssigkeit und dem Dampf aufrechterhalten wird. Dies ermöglicht es, eine Probe in einer dünnen Wasserschicht zu beobachten. Die Detektion erfolgt typischerweise über den sekundären Elektronendetektor, wobei diese Vorgehensweise jedoch nur für die Probenoberfläche möglich ist. Die Beobachtung von Schichtdicken von bis zu einhundert Nanometern innerhalb der Probe ist mit einem Rückstreu-Elektronendetektor möglich. Dieses letztere Detektionsverfahren ist jedoch relativ ineffizient und aufgrund der Strahlungsschäden nicht einsetzbar für die Untersuchung von nativem Zellmaterial. Zellen müssen fixiert und mit Metall eingefärbt werden, um die hierbei auftretenden Strahlungsschäden zu überstehen und damit während das Abbildens genügend Kontrast erzeugt wird. Eine viel effizientere Detektion spezifischer Marker, die aus Nanopartikeln hoher Atomzahlen bestehen, ist mit dem STEM-Detektor in den ausgebreiteten Zellregionen möglich. Nanopartikelmarker können mittels STEM effizient in ganzen Zellen detektiert werden, wobei nur eine minimale Probenvorbereitung erforderlich ist.

Von besonderem Interesse ist die Kombination von Lichtmikroskopie mit Elektronenmikroskopie, die sogenannte korrelative Mikroskopie. Zellvorgänge und Zellstrukturen werden dabei mit begrenzter Auflösung mittels Lichtmikroskopie beobachtet, während die hochauflösende Elektronenmikroskopie zu bestimmten Zeitpunkten und in bestimmten Regionen zur Anwendung kommt. Der Elektronenstrahl schädigt in der Regel das biologische Material. Die Lichtmikroskopie kann somit dazu verwendet werden, die Regionen und Zeitpunkte auszuwählen, die von besonderem Interesse sind, da hierdurch die Probe nicht geschädigt wird. Sie bringt auch wichtige zusätzliche Informationen für die Elektronenmikroskopie, zum Beispiel ermöglicht sie Überblickbilder der Zellen, Informationen über das Vorliegen bestimmter Proteine in bestimmten Zellregionen, beispielsweise unter Verwendung von spezifischen fluoreszierenden Markern. Marker können beispielsweise genetisch hergestellte Marker sein, wie das grüne fluoreszierende Protein oder Marker, die mit fluoreszierenden Atomen hoher Atomzahl dotiert sind, z.B. sogenannte "quantum dots". Im Prinzip werden Lichtmikroskopiebilder mit einer anderen Vorrichtungen als derjenigen, die für die Elektronenmikroskopie verwendet werden, aufgenommen, so daß die zeitliche Korrelation verlorengeht und der jeweilige abgebildete Bereich lokalisiert werden muß.

Es wäre äußerst vorteilhaft, wenn beide Mikroskopierverfahren in dem gleichen System durchgeführt werden könnten, so daß eine größtmögliche zeitliche Korrelation der Beobachtungen von Vorgängen und strukturellen Informationen gleichzeitig über beide Verfahren erreicht wird, wobei Fehler bezüglich der Lokalisierung von Bereichen oder die Veränderung von Strukturen über die Zeit vermieden werden.

Hierfür ist einerseits ein kombiniertes Fluoreszenzmikroskop, das auf einer hochvoltigen Transmissionselektronenmikroskop- (TEM; transmission electron microscopy) oder STEM-Säule angebracht ist, womit eine Zeitkorrelation im Bereich von etwa 30 Sekunden erreicht wird, indem die Probe zwischen dem senkrechten Elektronenstrahl und dem Lichtstrahl rotiert wird. Ein anderer Ansatz ist die Integration einer optischen Linse in ein ESEM-Mikroskop und Elektronenrückstreudetektion, welche jedoch relativ ineffizient und aufgrund der Strahlungsschäden nicht gut für empfindliche biologische Proben einsetzbar ist.

US 5,811,804 beschreibt eine Vorrichtung zur korrelierten Raster-Transmissionselektronenmikroskopie (STEM) und Raman-Spektroskopie.

WO 2010/120238A1 und US 4,990,776 beschreiben eine Vorrichtung zur Elektronenmikroskopie mit einem zusätzlichen optischen Detektionsweg.

US 2012/0120226A1 beschreibt eine mikrofluide Kammer für die Transmissionselektronenmikroskopie für lebende Zellen. Die erhaltenen Aufnahmen werden mit lichtmikroskopischen Aufnahmen korreliert.

EP 2 975 631 A1 und EP 2 924 706 A1, welche nach Art. 54(3) EPÜ als Stand der Technik gelten, offenbaren eine Vorrichtung zur korrelierten Elektronenmikroskopie und Lichtmikroskopie mit einem STEM-Detektor zwischen der Probe und der optischen Linse.

Die Aufgabe der vorliegenden Erfindung besteht darin, eine Vorrichtung für das korrelative Mikroskopieren gemäß dem Oberbegriff zu schaffen, die eine verbesserte Kombination von lichtmikroskopischem und STEM-Verfahren ermöglicht.

Diese Aufgabe wird durch die erfindungsgemäße Vorrichtung dadurch gelöst, daß ein STEM-Detektor in eine lichtoptische Linse integriert ist.

Diese Detektionsvorrichtung kombiniert die effiziente Detektion mittels STEM-Mikroskopie von Materialien mit hoher Atomzahl, beispielsweise spezifischen Nanopartikel-Markern in einer Probe in einer Flüssigkeit, wie einer Zelle, mit gleichzeitiger Lichtmikroskopie, beispielsweise über Fluoreszenzkontrast an fluoreszierenden Proteinmarkern in Zellen oder über Streukontrast des Zellmaterials. Die Erfindung hebt sich insofern vom Stand der Technik ab, als sie eine höchst effiziente Detektion von Materialien mit hoher Atomzahl in der Probe mit größtmöglicher Auflösung und eine vollständig zeitkorrelative Lichtmikroskopie ermöglicht. Eine Probe kann entweder getrocknet, in eine dünne Flüssigkeitsschicht eingebettet sein, oder in eine Eisschicht eingebettet sein.

In diesem Zusammenhang ist es vorteilhaft, daß der STEM-Detektor in einem Hohlraum in der lichtoptischen Linse angeordnet ist.

Erfindungsgemäß ist vorgesehen, daß der Hohlraum probenseitig eine Öffnung mit geringem Durchmesser aufweist, an die sich ein konisch ausgebildeter Elektronendriftraum anschließt, an dessen unteren Ende der STEM-Detektor angeordnet ist.

Unten bezieht sich hierbei auf eine Elektronenstrahlrichtung von oben nach unten. Beliebige Strahlenrichtungen sind möglich, abhängig von der Anordnung der Vorrichtung. Dies bedeutet, daß auf der der Probenhalterung zugewandten Seite der Linse die Öffnung angeordnet ist, an die sich der konisch nach unten erweiternde Elektronendriftraum anschließt, an dessen unteren Ende der STEM-Detektor angeordnet ist.

Es ist im Rahmen der Erfindung vorgesehen, daß das Signal des STEM-Detektors auf der Seite der Linse nach außen ausführbar ist.

Es ist im Rahmen der Erfindung vorgesehen, daß auf der der Probe zugewandten Seite der lichtoptischen Linse eine Probenhalterung angeordnet ist.

In diesem Zusammenhang ist vorgesehen, daß die Probenhalterung als dünne elektronendurchlässige Membran ausgebildet ist.

Da der Elektronenstrahl die auf der Probenhalterung angeordnete Probe durchdringt, muß die Probenhalterung elektronendurchlässig ausgebildet sein.

Weiterhin ist zur Erfindung gehörig, daß der Raum um die Probenhalterung und der Elektronendriftraum die Eigenschaft besitzen, ein Vakuum einzustellen.

Es muß die Möglichkeit bestehen, im Bereich der Probe und im Elektronendriftraum ein Vakuum zu erzeugen.

Weiterhin ist im Rahmen der Erfindung vorgesehen, daß eine Elektronenstrahlquelle auf der dem STEM-Detektor gegenüberliegenden Seite der der Probenhalterung angeordnet ist.

Weiterhin ist es erfindungsgemäß, daß eine Lichtquelle und lichtoptische Detektionsmittel mit der Linse verbunden sind.

Die Linse mit integriertem STEM-Detektor kann in unterschiedliche Elektronenmikroskope eingebaut werden, z.B. ein ESEM mit einer typischen Elektronenenergie von 30 keV oder ein hochauflösendes STEM mit einer typischen Elektronenenergie von 200 keV.

Es ist zur Erfindung gehörig, daß ein oder mehrere andere lichtoptische Strahlengänge zur Detektion oder zur Beleuchtung vorgesehen sind.

Eine weitere Ausgestaltung der Erfindung sieht vor, daß die Lichtquelle seitlich des STEM-Detektors angeordnet ist, der Lichtstrahl sich im Fokus mit dem Elektronenstahl überlappt und ein lichtoptischer Detektionsweg sich mit dem Beleuchtungsstrahl überlappt.

Es liegt im Rahmen der Erfindung, daß der STEM-Detektor eine oder mehr als eine Detektionsfläche aufweist, wovon mindestens eine die gestreuten Elektronen auffängt.

Ein mögliches Anwendungsgebiet der Erfindung ist die Untersuchung von Zellvorgängen, die auf dem dynamischen Aufbau und Abbau von Proteinkomplexen beruhen, wobei eine Kombination von verschiedenen Nanopartikeln verwendet wird, um eine Vielzahl von verschiedenen Proteinsorten zu detektieren, welche mit verschiedenen fluoreszierenden proteinspezifischen Markern kombiniert werden. Ein spezifisches Forschungsgebiet kann beispielsweise die biomedizinische Forschung, insbesondere die Krebsforschung sein. Ein anderes mögliches Anwendungsgebiet der Erfindung ist die Untersuchung der Interaktion von Nanomaterialien mit Zellen, wobei Übersichtsbilder von Zellen erhalten werden können und auch die Verwendung von fluoreszierenden Markern, um Bereiche mit gewissen Proteinen, bestimmten Organellen, Lipiden, DNS, etc. aufzuzeigen. Weitere Anwendungsgebiete sind die Pathologie und Gerichtsmedizin, die Untersuchung von Meeresorganismen und das Abbilden von Bakterien und Viren. Schließlich kommt auch die Diagnose der Anwesenheit von spezifischen Proteinen oder anderen Makromolekülen und bestimmten Protein- und Makromolekülansammlungen, beispielsweise zum Detektieren von Krebs in Zellmaterial in Betracht.

Weiterhin kann die Erfindung auch Anwendung finden bei der Untersuchung von Proben aus anderen Bereichen, beispielsweise den Materialwissenschaften, wobei eine Kombination von Lichtmikroskopie und Elektronenmikroskopie benötigt wird, beispielsweise auf dem Gebiet der Energiespeicherung unter Verwendung von Batteriematerialien. Weitere mögliche Anwendungsgebiete sind die Untersuchung von Polymeren, die Charakterisierung von Nanopartikeln, das Testen von feinmechanischen Werkzeugen und das Abbilden von unterschiedlichen Materialien, beispielsweise Fragmenten aus korrodierten Stahlrohren. Nachfolgend werden einige Ausführungsbeispiel der Erfindung anhand von Zeichnungen erläutert.

Es zeigen
- Fig. 1: eine schematische Darstellung einer erfindungsgemäßen Detektionsvorrichtung,
- Fig. 2: ein schematisches Detail der Detektionsvorrichtung für die korrelative Raster-Transmissionselektronenmikroskopie und Lichtmikroskopie,
- Fig. 3: eine schematische Darstellung eines nicht erfindungsgemäßen Beispiels einer nicht erfindungsgemäßen Detektionsvorrichtung,
- Fig. 4: ein schematisches Detail eines nicht erfindungsgemäßen Beispiels einer Detektionsvorrichtung für die korrelative Raster-Transmissionselektronenmikroskopie und Lichtmikroskopie.

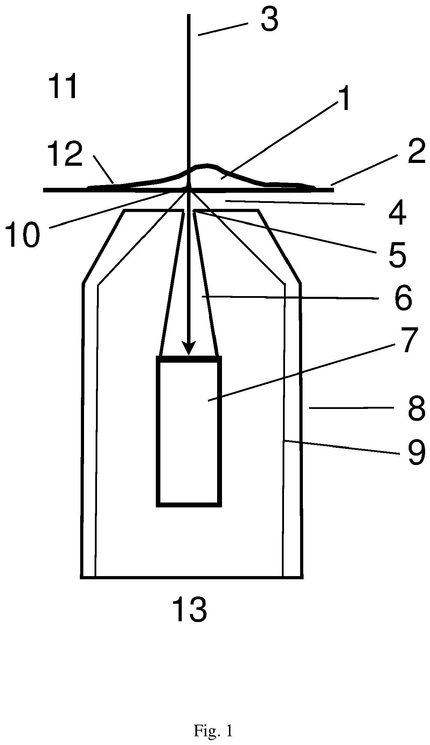

Fig. 1 zeigt eine Detektionsvorrichtung für die korrelative Raster-Transmissionselektronenmikroskopie und Lichtmikroskopie. Eine Probe (1), beispielsweise eine eukaryotische Zelle (1) ist auf einer dünnen elektronendurchlässigen Membran (2) angeordnet. Die Membran (2) ist mit einer Probenhalterung verbunden. Die Probe (1) wird mit einem Elektronenstrahl (3) abgebildet, wobei typischerweise die Probe (1) mit einem Elektronenstrahl (3) abgescannt wird. Übertragene Elektronen propagieren durch die Membran (2) über einen Spalt (4) in einen Hohlraum mit kleiner Öffnung (5) in einen Elektronendriftraum (6) zum STEM-Detektor (7). Der Elektronendriftraum (6) und der STEM-Detektor (7) sind in einem Hohlraum in der lichtoptischen Linse (8) angeordnet. Die Linse (8) fokussiert einen breiten Lichtstrahl (9) in einem Fokalpunkt (10) auf die Probe (1). Die Probe (1) und der Elektronendriftraum (6) befinden sich im Vakuum (11). Eine dünne Flüssigkeitsschicht (12) wird über der Probe (1) aufrechterhalten. Die lichtoptische Detektion und die Lichtquelle sind in dem Bereich (13) unter der Linse (8) angeordnet.

Die erfindungsgemäße Vorgehensweise ist wie folgt.

Eine Probe (1) in einer Flüssigkeit, beispielsweise eine eukaryotische Zelle, ist auf einer dünnen Membran (2) in dem Elektronenmikroskop angeordnet. Die Membran (2) besteht aus leichten Materialien mit geringer Atomzahl, beispielsweise Kohlenstoffnitrid oder Siliziumnitrid, so daß ein Elektronenstrahl (3) mit ausreichender Energie, beispielsweise 30 keV oder 200 keV, die Membran (2) durchdringen kann.

Die Membran (2) wird von einem Träger, z.B. einem Siliziummikrochip oder einem dünnen Material, getragen. Der Träger ist auf einer Probenhalterung mit Mitteln zum Bewegen in x, y und z-Richtung angeordnet. Der Elektronenstrahl (3) ist so konfiguriert, daß die Strahlrichtung von der Oberseite zu der Unterseite des Mikroskops und durch die Probe (1) verläuft. Selbstverständlich kann der Elektronenstrahl statt von oben nach unten auch in einer anderen Richtung verlaufen, beispielsweise von unten nach oben oder von links nach rechts.

Eine spezielle optische Linse (8) ist unter der Membran (2) angeordnet. Der Strahlengang ist so angeordnet, daß das Licht durch die Linse (8) tritt und auf die Probe fokussiert wird, während reflektiertes oder fluoreszierendes Licht mit der gleichen Linse (8) gesammelt und zum Detektor geleitet wird, beispielsweise unter Verwendung eines optischen Filterwürfels, der aus zweifarbigen Spiegeln besteht. Der Lichtstrahl konvergiert in Richtung der Probe (1) mit einem Öffnungshalbwinkel von beispielsweise 0,75 rad, der durch die numerische Öffnung der Linse (8) bestimmt wird. Eine hochauflösende Luft- oder Vakuumlinse ermöglicht eine 100-fache Vergrößerung bei einer numerischen Öffnung von 1,0 und einer Arbeitsdistanz von 0,15 mm. Die Linse (8) fokussiert den Lichtstrahl (9) auf die Probe (1), wobei ein breiter Lichtstrahl (9) mit einem Durchmesser von beispielsweise 9 mm am anderen Ende der Linse (8) vorliegt. Die Linse (8) weist eine konische Vertiefung auf, die entlang der optischen Achse der Linse (8) angeordnet ist und als Elektronendriftraum (6) dient. Die Maße der konischen Vertiefung passen zu dem Strahlengang, der für die STEM-Detektion verwendet wird mit typischen Öffnungshalbwinkeln von 0,050 bis 0,20 rad. Der Durchmesser der Vertiefung an der Oberseite der Linse (8) direkt unter der Probe (1) beträgt beispielsweise 0,060 mm, der um 0,20 rad bei einem Abstand von 0,15 mm abnimmt. Die Vertiefung erstreckt sich durch die Linse (8) mit einem Winkel von 0,2 rad. Bei einer Linse (8) von 10 mm Länge beträgt die Vertiefung an der Unterseite der Linse (8) somit 4,0 mm. Der STEM-Detektor (7) wird an der weitesten Stelle der Vertiefung angeordnet.

Obwohl die Anwesenheit der Vertiefung den Strahlengang durch die Linse (8) beschränkt, reduziert sie lediglich die Gesamtmenge an Licht, das durch die Linse (8) durchtritt, wobei die Linse (8) immer noch die Fähigkeit hat, den Lichtstrahl auf die Probe (1) zu fokussieren, Licht von der Probe zu sammeln und für die Lichtmikroskopie ein vergrößertes Bild des beleuchteten Bereichs auf einen positionssensitiven Lichtdetektor zu projizieren. Alternativ kann ein konfokales optisches System verwendet werden. Alternativ können auch mehrere zusätzliche lichtoptische Strahlengänge zur Detektion oder Beleuchtung benutzt werden.

Der STEM-Detektor besteht aus einem Zylinder aus Szintillatormaterial von einem Durchmesser von beispielsweise 4,0 mm, um Elektronen in Lichtpulse umzuwandeln. Der Szintillator ist mit einem empfindlichen Lichtdetektor gekoppelt, beispielsweise einer Photovervielfacher-Röhre. Diese Röhre ist seitlich an der Linse angeschlossen. Dazu hat der Szintillator seitlich der optischen Linse eine Anschlußfläche. Die Außenseite ist mit reflektierendem und leicht abgestuftem Material versehen, so daß das Licht aus dem lichtoptischen Strahlengang nicht in den STEM-Detektor (7) eingekoppelt wird. Der STEM-Detektor (7) kann auch aus einem kleinen Mikrochip mit einem ladungsgekoppelten Elementdetektor bestehen. Der Mittelkreis des STEM-Detektors (7) mit einem entsprechenden Halbwinkel von 50 mrad ist von der Detektion entkoppelt, beispielsweise blockiert oder das Signal aus diesem Bereich wird als sekundäres Detektionssignal verwendet.

So registriert der STEM-Detektor (7) Signale von übermittelten Elektronen mit Halbwinkeln von 50 mrad bis 0,20 rad. Das Signal wird als Dunkelfeldsignal bezeichnet.

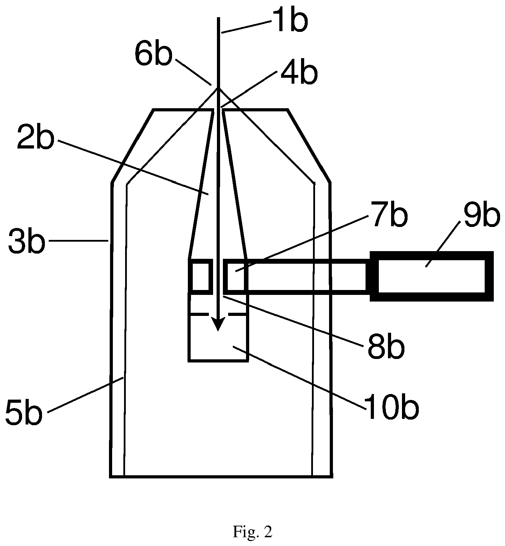

Fig. 2 zeigt ein schematisches Detail der Detektionsvorrichtung für die korrelative Raster-Transmissionselektronenmikroskopie und Lichtmikroskopie. Ein durch eine Probe (hier nicht angezeigt) transmittierter Elektronenstrahl (1b) kommt in einen Elektronendriftraum (2b), der in einem Hohlraum in einer lichtoptischen Linse (3b) angeordnet ist. Der Elektronendriftraum ist durch eine kleine Öffnung (4b) zugänglich. Die Linse (3b) fokussiert einen breiten Lichtstrahl (5b) in einem Fokalpunkt (6b). Im Elektronendriftraum (2b) befindet sich ein STEM-Detektor (7b), welcher hergestellt ist aus einem Stab aus szintillierendem Material, damit Elektronen in Lichtpulse umgewandelt werden. Der Stab (7b) hat eine Öffnung (8b), damit Elektronen im Hellfeld den Detektor ungehindert passieren und nur Elektronen, die um einen bestimmten Kleinstwinkel gestreut sind, detektiert werden. Der stabförmige STEM-Detektor (7b) führt seitlich aus der Linse (3b) heraus und ist verbunden mit einem empfindlichen Lichtdetektor, so wie einem Photomultiplier oder einer Photodiode (9b), um das Licht in ein elektrisches Signal umzuwandeln. Statt eines Stabs aus szintillierendem Material und einem Lichtdetektor kann auch ein anderer Elektronendetektor wie zum Beispiel ein Halbleiter-Elektronen-Detektor, der von vergleichbaren Abmessungen ist, verwendet werden. Elektronen im Hellfeld werden in einer separaten Kammer mit kleiner Öffnungsblende (10b) eingefangen, damit sie nicht das STEM-Signal stören.

In einem weiteren, nicht erfindungsgemäßen, Beispiel wird der STEM-Detektor nicht in der optischen Linse, sondern in der Nähe der optischen Linse montiert. Der STEM-Detektor fängt den Elektronenstrahl auf. Der lichtoptische Strahl ist derart gelenkt, daß dieser im Fokus mit dem Elektronenstrahl überlappt, aber ansonsten seitlich des Elektronenstrahls verläuft. Der STEM-Detektor kann seitlich an der lichtoptischen Linse angeordnet sein. Das lichtoptische System kann auch aus mehreren Strahlengängen und Linsen bestehen.

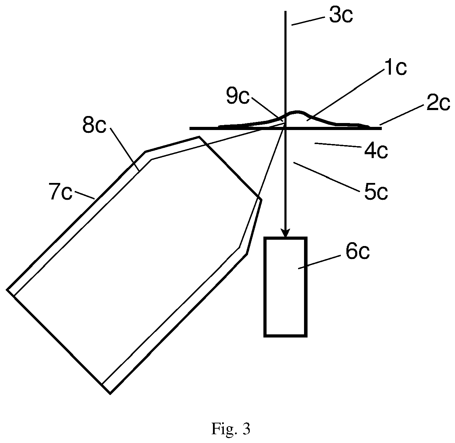

Fig. 3 zeigt ein weiteres nicht erfindungsgemäßes Beispiel einer Detektionsvorrichtung für die korrelative Raster-Transmissionselektronenmikroskopie und Lichtmikroskopie. Eine Probe (1c), beispielsweise eine eukaryotische Zelle (1c), ist auf einer dünnen elektronendurchlässigen Membran (2c) angeordnet. Die Membran (2c) ist mit einer Probenhalterung verbunden. Die Probe (1c) wird mit einem Elektronenstrahl (3c) abgebildet. Elektronen propagieren durch die Membran (2c) und verlaufen im Vakuum unterhalb der Membran (4c). Der transmittierte Elektronenstrahl (5c) propagiert weiter bis zum STEM-Detektor (6c). Eine lichtoptische Linse (7c) ist seitlich des Elektronstrahls (5c) angeordnet. Die Linse (7c) fokussiert einen breiten Lichtstrahl (8c) in einem Fokalpunkt (9c) auf der Probe (1c). Die lichtoptische Detektion und die Lichtquelle sind unterhalb der Linse (7c) angeordnet.

In einem weiteren nicht erfindungsgemäßen Beispiel werden ein dünner STEM-Detektor zwischen der lichtoptischen Linse und der Probe positioniert. In Abhängigkeit von der Konfiguration des STEM-Detektors kann zeitnah das Lichtsignal und das STEM-Signal detektiert werden. Sobald der STEM-Detektor aus dem Lichtstrahlengang herausgefahren wird, kann die lichtoptische Detektion erfolgen.

In einem weiteren Ausführungsbeispiel ist der STEM-Detektor nicht symmetrisch ausgebildet, sondern hat eine oder mehr als eine Detektionsfläche, wovon mindestens eine die gestreuten Elektronen auffängt. Die Detektorflächen können innerhalb, oberhalb oder seitlich der lichtoptischen Linse angebracht werden. Das Lichtsignal und das STEM-Signal können gleichzeitig detektiert werden

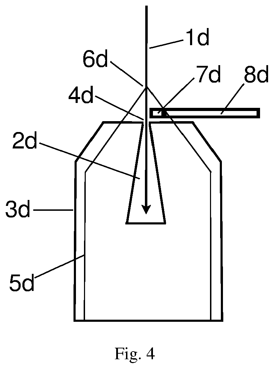

Fig. 4 zeigt ein schematisches Detail eines nicht erfindungsgemäßen Beispiels einer Detektionsvorrichtung für die korrelative Raster-Transmissionselektronenmikroskopie und Lichtmikroskopie. Ein durch eine Probe (hier nicht angezeigt) transmittierender Elektronenstrahl (1d) wird in einen Hohlraum (2d) in einer lichtoptische Lise (3d) aufgefangen. Der Hohlraum (2d) ist durch eine kleine Öffnung (4d) zugänglich. Die Linse (3d) fokussiert einen breiten Lichtstrahl (5d) in einem Fokalpunkt (6d) auf der Probe. Zwischen Fokalpunkt (6d) und Linsenoberkante mit kleiner Öffnung (4d) befindet sich eine STEM-Detektionsfläche (7d). Die STEM-Detektionsfläche (7d) kann aus einen Stab aus szintillierendem Material hergestellt werden und dieser wird an einen Lichtdetektor (8d) angeschlossen. Es kann auch ein Halbleiter-Elektronendetektor benutzt werden.

Ein Anwendungsbeispiel für ein erfindungsgemäßes Verfahren wird im folgenden beschrieben. Als Beispiel wird eine eukaryotische Zelle, beispielsweise eine COS7 Fibroblastenzelle, verwendet. Die Zelle enthält Nanopartikel, beispielsweise Goldnanopartikel von 5 nm Durchmesser. Die Nanopartikel weisen eine Beschichtung für das spezifische Anbinden des Nanopartikels an ein Protein auf, beispielsweise eine Beschichtung, die ein Molekül mit epidermalen Wachstumsfaktoren enthält. Dieses Molekül ist ein Ligand für den Rezeptor für epidermale Wachstumsfaktoren. Die Detektion dieses Rezeptors ist wichtig für die Forschung über und die Diagnose von bestimmten Krebsarten, beispielsweise Brustkrebs. Die Zelle wird in Flüssigkeit auf einer Stützmembran immobilisiert und mittels Lichtmikroskopie untersucht. Verschiedene Regionen der Zelle können durch Bewegen der Probenhalterung untersucht werden. Der Fokus wird angepaßt, indem die Zelle in z-Richtung bewegt wird. Zu einem bestimmten Zeitpunkt werden Nanopartikel auf die Probe aufgebracht, wobei die Probe weiter mittels Lichtmikroskopie untersucht wird. Wenn ein besonders interessanter Bereich vorliegt, wird die Luft um die Probe im Wesentlichen mittels einer Vakuumpumpe abgesaugt und die Temperatur der Probe auf ein paar Grad Celsius abgesenkt. Dann wird der Druck so geregelt, daß ein Gleichgewicht zwischen dampfförmigem und flüssigem Wasser vorliegt. Auf diese Weise wird erreicht, daß ein dünner Wasserfilm über der Probe erhalten bleibt, während das Innere der Zelle Wasser enthält. In bestimmten Fällen kann das Zellmaterial fixiert werden, beispielsweise mittels Glutaraldehyd, und die Flüssigkeit durch reines Wasser ersetzt werden. Der Elektronenstrahl wird dann angeschaltet und mit diesem wird die Probe gescannt. Der Elektronenstrahl ist ein dünner konvergierender Strahl mit einem typischen Halbwinkel von 5 mrad und er wird auf einen besonders interessierenden Punkt der Probe gerichtet. Es wird ein Kontrast auf dem Zellmaterial erzeugt, indem der Elektronenstrahl gestreut wird. Gestreute und in einem Konus mit einem Öffnungshalbwinkel von 0,20 mrad werden in dem Driftraum aufgenommen und gelangen zum Detektor. Die Signale wenigstens der Elektronen mit Halbwinkeln zwischen 50 mrad und 0,20 rad werden mit dem STEM-Detektor gesammelt. Pixel für Pixel wird der Elektronenstrahl über die Probe gescannt und das STEM-Signal gespeichert. Mit STEM werden eine Auflösung im Nanometerbereich, ein hoher Kontrast bei Markern mit hoher Atomzahl und eine kleinere Auflösung im Bereich des Zellmaterials erreicht. Der lichtoptische Strahl wird gleichzeitig verwendet, um die Probe auszuleuchten und entweder gestreutes oder fluoreszierendes Licht zu sammeln. Lichtmikroskopie kann auch nach der STEM-Detektion verwendet werden, um die Zelle zu untersuchen, nachdem sie dem Elektronenstrahl ausgesetzt war und um verschiedene Zellregionen zu untersuchen.

Im Rahmen der vorliegenden Erfindung liegt ein Verfahren, um eine Probe mittels Licht- und Elektronenmikroskopie zu untersuchen, wobei die Licht- und Elektronenstrahlen räumlich und zeitlich verbunden bzw. korreliert sind. Dazu wird zuerst die Probe auf einen dünnen Träger montiert und dieser in x-, y- und z-Richtung auf die gewünschte Position relativ zu dem Elektronenstrahl gefahren. Die räumliche Positionierung des Elektronenstrahls, des Lichtstrahls und der Probe sind genauestens bekannt, somit sind die Bilder entsprechend räumlich korreliert. Hierzu wird das Gerät exakt dimensioniert. Zusätzlich kann der räumliche Zusammenhang bzw. die Korrelation der Strahlengänge kalibriert werden. Hierfür werden Aufnahmen für Bilder mit gut erkennbaren Objekten verwendet. Die Korrelation der Strahlengänge kann daraus ermittelt werden und durch Speicherung wird der räumliche Zusammenhang der Bilder von zu untersuchenden Proben ermöglicht. Nachdem die Bilder aufgenommen sind, werden diese beispielsweise in einer Bilddatei gespeichert, wodurch diese mit den räumlichen Korrelationsdaten gekoppelt werden können. Bilder der Licht- und Elektronenmikroskopie können entweder getrennt oder aber im selben Koordinatensystem dargestellt bzw. überlagert werden, damit ein Gesamtbild der Probe entsteht.

Die Erfindung umfaßt auch ein Verfahren, um die licht- und elektronenmikroskopischen Bilder zeitlich korrelieren zu können. Dazu werden beispielsweise die licht- und elektronenmikroskopischen Bilder gleichzeitig oder fast gleichzeitig aufgenommen und deren Zeitpunkt und Dauer der Aufnahme gemessen oder berechnet. So werden z.B. die beiden Bilder der Licht- und der Elektronenmikroskopie und gleichzeitig die Zeitpunkte der Aufnahmen gespeichert. Die Aufnahme der Bilder kann auch in einer bestimmten Reihenfolge stattfinden, z.B. zwei lichtmikroskopische Bilder und ein STEM-Bild. Die Aufnahme der Bilder kann auch repetitiv erfolgen. Beispielsweise können 100 lichtmikroskopische Bilder mit dazwischen liegenden kurzen Pausen aufgenommen werden, während parallel über das STEM-Verfahren sieben Bilder aufgenommen werden. Bei diesem Verfahren werden die Bilder einschließlich des Zeitpunktes und der Dauer der Aufnahmen gespeichert, wodurch eine zeitlich korrelierte Darstellung der Bilder möglich ist.

## Patentansprüche

1. Vorrichtung für die korrelative Raster-Transmissionselektronenmikroskopie - STEM - und Lichtmikroskopie zur Untersuchung einer Probe (1) umfassend einen STEM-Detektor, **dadurch gekennzeichnet, daß** der STEM-Detektor (7) in eine lichtoptische Linse (8) integriert ist.

2. Vorrichtung gemäß Anspruch 1, **dadurch gekennzeichnet, daß** der STEM-Detektor in einem Hohlraum (5) in der lichtoptischen Linse (8) angeordnet ist.

3. Vorrichtung gemäß Anspruch 2, **dadurch gekennzeichnet, daß** der Hohlraum (5) probenseitig eine Öffnung mit geringem Durchmesser aufweist, an die sich ein konisch ausgebildeter Elektronendriftraum (6) anschließt, an dessen unteren Ende der STEM-Detektor (7) angeordnet ist.

4. Vorrichtung gemäß einem der Ansprüche 1 bis 3, **dadurch gekennzeichnet, daß** das Signal des STEM-Detektors (7) auf der Seite der Linse nach außen ausführbar ist.

5. Vorrichtung gemäß einem der Ansprüche 1 bis 4, **dadurch gekennzeichnet, daß** auf der der Probe (1) zugewandten Seite der lichtoptischen Linse (8) eine Probenhalterung (2) angeordnet ist.

6. Vorrichtung gemäß Anspruch 5, **dadurch gekennzeichnet, daß** die Probenhalterung (2) als dünne elektronendurchlässige Membran (2) ausgebildet ist.

7. Vorrichtung gemäß einem der Ansprüche 5 oder 6, **dadurch gekennzeichnet, daß** eine Elektronenstrahlquelle auf der dem STEM-Detektor (7) gegenüberliegenden Seite der Probenhalterung (2) angeordnet ist.

8. Vorrichtung gemäß einem der Ansprüche 1 bis 7, **dadurch gekennzeichnet, daß** eine Lichtquelle und lichtoptische Detektionsmittel mit der Linse (8) verbunden sind.

9. Vorrichtung gemäß Anspruch 8, **dadurch gekennzeichnet, daß** ein oder mehrere andere lichtoptische Strahlengänge zur Detektion oder zur Beleuchtung vorgesehen sind.

10. Vorrichtung gemäß einem der Ansprüche 8 oder 9, **dadurch gekennzeichnet, daß** die Lichtquelle seitlich des STEM-Detektors (7) angeordnet ist, der Lichtstrahl sich im Fokus mit dem Elektronenstahl (3) überlappt und ein lichtoptischer Detektionsweg sich mit dem Beleuchtungsstrahl überlappt.

## Claims

1. Device for correlative scanning transmission electron microscopy - STEM - and light microscopy for the investigation of a specimen (1) comprising a STEM detector, **characterized in that** the STEM detector (7) is integrated in a photo-optical lens (8).

2. Device according to claim 1, **characterized in that** the STEM detector is positioned in a cavity (5) in the photo-optical lens (8).

3. Device according to claim 2, **characterized in that** the cavity (5) has, at the specimen end, a small-diameter opening followed by a conical electron drift chamber (6) at the lower end of which the STEM detector (7) is located.

4. Device according to any one of the claims 1 to 3, **characterized in that** the signal from the STEM detector (7) can be transmitted to the outside at the side of the lens.

5. Device according to any one of the claims 1 to 4, **characterized in that** a specimen holder (2) is located at that end of the photo-optical lens (8) which is nearer to the specimen (1).

6. Device according to claim 5, **characterized in that** the specimen holder (2) is configured as thin, electron-permeable membrane (2).

7. Device according to any one of the claims 5 or 6, **characterized in that** an electron beam source is located on the other side of the specimen holder (2) from the STEM detector (7).

8. Device according to any one of the claims 1 to 7, **characterized in that** a light source and photo-optical detection means are connected to the lens (8).

9. Device according to claim 8, **characterized in that** one or a plurality of other photo-optical beam paths are provided for purposes of detection or illumination.

10. Device according to either of the claims 8 or 9, **characterized in that** the light source is fitted at the side of the STEM detector (7), the focus of the light beam overlaps with the electron beam (3) and a photo-optical detection path overlaps with the illumination beam.

## Revendications

1. Dispositif destiné à la microscopie corrélative au moyen d'un microscope électronique à balayage en transmission, MEBT, et à la microscopie optique pour l'examen d'un échantillon (1), comprenant un détecteur MEBT, **caractérisé en ce que** le détecteur MEBT (7) est intégré dans une lentille optique (8).

2. Dispositif selon la revendication 1, **caractérisé en ce que** le détecteur MEBT est disposé dans une cavité (5) dans la lentille optique (8).

3. Dispositif selon la revendication 2, **caractérisé en ce que** la cavité (5) présente côté échantillon une ouverture de diamètre réduit, prolongée par un espace de dérive d'électrons (6) à l'extrémité inférieure duquel est disposé le détecteur MEBT (7).

4. Dispositif selon l'une quelconque des revendications 1 à 3, **caractérisé en ce que** le signal du détecteur MEBT (7) peut être amené à l'extérieur du côté de la lentille.

5. Dispositif selon l'une quelconque des revendications 1 à 4, **caractérisé en ce qu'**un porte-échantillon (2) est disposé sur le côté tourné vers l'échantillon (1) de la lentille optique (8).

6. Dispositif selon la revendication 5, **caractérisé en ce que** le porte-échantillon (2) est réalisé comme une fine membrane (2) perméable aux électrons.

7. Dispositif selon l'une quelconque des revendications 5 et 6, **caractérisé en ce qu'**une source de faisceaux d'électrons est disposée sur un côté du porte-échantillon (2) opposé au détecteur MEBT (7).

8. Dispositif selon l'une quelconque des revendications 1 à 7, **caractérisé en ce qu'**une source de lumière et des moyens de détection optiques sont reliés à la lentille (8).

9. Dispositif selon la revendication 8, **caractérisé en ce qu'**un ou plusieurs chemins de rayons optiques sont prévus pour la détection ou pour l'éclairage.

10. Dispositif selon l'une quelconque des revendications 8 et 9, **caractérisé en ce que** la source de lumière est disposée sur le côté du détecteur MEBT (7), le faisceau de lumière se chevauche avec le faisceau d'électrons (3) au niveau du foyer, et un chemin de détection optique se chevauche avec le faisceau d'éclairage.
